# EUROPÄISCHE PATENTSCHRIFT

(11) **EP 2 070 555 B1**
(45) Veröffentlichungstag und Bekanntmachung des Hinweises auf die Patenterteilung: **20.02.2013**
(21) Anmeldenummer: 08021699.7
(22) Anmeldetag: 15.12.2008
(51) Int. Cl.: A61L 15/14, A61L 17/14, A61L 31/14, A61L 31/08, A61F 2/00

(54) **Fluorirtes Implantat**
Fluorinated implant
Implant fluoré

(30) Priorität: 14.12.2007 DE 102007062273
(43) Veröffentlichungstag der Anmeldung: 17.06.2009
(73) Patentinhaber: Aesculap AG, 78532 Tuttlingen (DE)
(72) Erfinder: Siedle, Gabriel, 79117 Freiburg (DE)
(74) Vertreter: Patentanwälte Ruff, Wilhelm, Beier, Dauster & Partner

(56) Entgegenhaltungen:
- EP-A1- 1 236 761
- WO-A1-2007/107261
- WO-A2-2007/025293
- DE-A1- 10 353 930
- DE-A1- 19 912 648
- W.W.VRIJLAND ET AL: "Peritoneal adhesions to prosthetic material" SURGICAL ENDOSCOPY, Bd. 14, 22. August 2000 (2000-08-22), Seiten 960-963, XP002577083
- Y.MAROIS ET AL.: "Biostability, Inflamators Response, and Healing Characteristics of a Fluoropassivated Polyester-Knit Mesh in the Repair of Experimental Abdominal Hernias" ARTIFICIAL ORGANS, Bd. 24, Nr. 7, 2000, Seiten 533-543, XP002577084
- B.M.SOARES ET AL.: "In vitro characterization of a fluoropassivated gelatin-impregantes polyester mesh for hernia-repair" JOURNAL OF BIOMEDICAL MATERIALS RESEARCH, Bd. 32, 1996, Seiten 259-270, XP002577085
- DATABASE WPI Week 200417 Thomson Scientific, London, GB; AN 2004-172004 XP002577086 & JP 2003 225311 A (NAKAJIMA N) 12. August 2003 (2003-08-12)
- R,D,SANDERSON ET AL.: "FLUORINATION RATES OF POLYOLEFINS AS A FUNCTION OF STRUCTURE AND GAS ATMOSPHERE" JOURNAL OF THERMAL ANALYSIS, Bd. 41, 1994, Seiten 563-581, XP002577087

## Beschreibung

Die vorliegende Erfindung betrifft ein medizinisches Implantat, welches sich insbesondere zur Versorgung von Hernien eignet.

Die Versorgung einer Hernie bzw. eines Eingeweidebruchs gehört zu den wichtigsten Aufgabenbereichen in der visceralen oder parietalen Chirurgie. Hierbei handelt es sich im Allgemeinen um einen Austritt von Eingeweiden aus der Bauchhöhle durch eine angeborene oder erworbene Öffnung, die sogenannte Bruchpforte. Unter den äußeren Brüchen, bei denen stets der Bruchsack vom Bauchfell umschlossen wird, sind Leisten-, Narben- und Nabelbrüche die häufigsten Formen. Ursachen für das Auftreten von Hernien sind vor allem Muskel- oder Bindegewebsschwächen. Diese können infolge von Überlastungen, altersbedingter Erschlaffung, ungenügender Narbenbildung nach einem operativen Eingriff oder einer angeborenen Schwächung der Bauchdecke entstehen.

Eine zielführende Behandlung sieht in den meisten Fällen einen operativen Eingriff vor, bei welchem der Bruchinhalt aus dem Bruchsack in den Bauch zurückverlagert und die Bruchpforte verschlossen wird. Der Verschluss der Bruchpforte kann beispielsweise mit Hilfe von Nahtmaterialien durchgeführt werden. Nachteilig hierbei ist jedoch, dass Nahtmaterialien im Allgemeinen nur begrenzt mechanischen Beanspruchungen, wie sie beispielsweise beim Husten auftreten, ausgesetzt werden können. Die Versorgung von Hernien allein mit Hilfe chirurgischer Nahtmaterialien verursacht daher nicht selten ein Bruchrezidiv.

Deshalb werden mittlerweile zunehmend künstliche Verstärkungsmaterialien in Form von textilen Netzen zur Bauchwandrekonstruktion eingesetzt. Diese Netze haben den Vorteil, dass sie in zwei oder mehr Richtungen Kräfte aufnehmen können und hierdurch eine Entlastung der eigentlichen Naht bewirken. Handelsübliche Netze bestehen überwiegend aus mono- oder multifilen Polymeren, insbesondere aus Polyester oder Polypropylen.

Allerdings treten bei der Verwendung von Hernienimplantaten der oben genannten Art häufig postoperative Komplikationen auf. Diese Komplikationen beruhen vor allem auf post-chirurgischen Verwachsungen mit dem Bauchraum. Dadurch kann die Beweglichkeit des Patienten dauerhaft eingeschränkt werden. Häufig sind derartige Verwachsungen auch schmerzhaft für den Patienten. Insgesamt besteht aber vor allem ein erhöhtes Risiko einer erneuten chirurgischen Intervention.

Daher sind inzwischen auch Hernienimplantate kommerziell erhältlich, die aufgrund ihrer besonderen Struktur Gewebeadhäsionen, insbesondere nach einer intraperitonealen Implantation, vorbeugen sollen. Dies kann beispielsweise durch eine einseitig mikroporöse Strukturierung des Implantats erreicht werden. Durch eine derartige Struktur kann eine zelluläre Besiedelung und damit das Eindringen von Körperzellen auf der mikroporösen Seite des Implantats im Wesentlichen vermieden werden. Ein Beispiel für ein solches Implantat ist aus der DE 199 12 648 A1 bekannt und wurde von der Anmelderin selbst entwickelt.

Bei den weiterhin zum Einsatz kommenden Implantaten handelt es sich insbesondere um kompositäre Implantate, die auf der Implantatseite, welche nach der Implantation dem Bauchraum zugewandt sein soll, gewebeadhäsionsbeständige Beschichtungen aufweisen. Beispielsweise sind derartige Hernienimplantate aus den EP 0 797 962 B1, EP 1 317 227 B1 und EP 0 998 314 B1 bekannt. Die Beschichtungen liegen häufig in Form von Filmen oder Folien vor. Beispielsweise geht ein mit einer Polymerfolie beschichtetes Herniennetz aus der DE 602 09 787 T2 hervor. Filme bzw. Folien neigen jedoch zu einer gewissen Brüchigkeit. Hierdurch kann es letztendlich doch zu unerwünschten Gewebeinfiltrationen des Implantats kommen, wodurch die bereits erwähnten post-chirurgischen Gewebeverwachsungen auftreten können.

In der EP 1 200 010 B1 wird eine Hernie-Prothese mit einer Sperrschicht aus einem Fluorpolymer beschrieben. Bei dem Sperrmaterial kann es sich insbesondere um Polytetrafluorethylen, fluoriertes Ethylenpropylen, Tetrafluorethylen, Ethylentetrafluorethylen sowie andere geeignete Fluorpolymere handeln. Ein weiteres Hernienimplantat mit monofilen Fäden aus Polyvinylidenfluorid oder einem hiervon abgeleiteten Derivat ist in der EP 1 411 997 B1 beschrieben. Nachteilig bei einem Implantat dieser Art ist sein allgemein höheres Flächengewicht im Vergleich zu beispielsweise reinen Polypropylen-Netzen. Daneben ist die Herstellung von Implantaten auf der Basis von Fluorpolymeren teuer, was insbesondere auf die hochpreisigen Fluor-Monomere, die zur Herstellung der Fluorpolymere verwendet werden, zurückzuführen ist.

W.Vrijland et al. in SURGICAL ENDOSCOPY, 14, 2000, 960-3; Y.Marois et al. in ARTIFICIAL ORGANS, 24(7), 2000 , 533-43 und B.M.Soares et al. in JOURNAL OF BIOMEDICAL MATERIALS RESEARCH, 32, 1996, 259-70 beschreiben mit fluorierten Polymeren beschichtete Polyester-Implantatnetze. JP2003225311 beschreibt mit Gasphasenfluorierung behandelte medizinische Gegenstände.

Die vorliegende Erfindung stellt sich daher die Aufgabe, ein medizinisches Implantat bereitzustellen, welches sich insbesondere für eine intraperitoneale Implantation eignet, ohne dass es hierbei zu post-chirurgischen Gewebeverwachsungen kommt. Das Implantat soll weiterhin einfach und insbesondere kostengünstig herstellbar sein.

Diese Aufgabe wird gelöst durch ein medizinisches Implantat aus an sich fluorfreien Polymeren, insbesondere zur Versorgung von Hernien, wobei das Implantat zumindest teilweise Fäden aufweist, die an ihrer Oberfläche zumindest teilweise eine fluorhaltige Schicht aufweisen, dadurch gekennzeichet, dass in der Fluorhaltigen Schicht Fluor kovalent an das Polymer des Implantats gebunden ist und das Implantat ein textiles Implantat ist.

Durch die Erfindung wird ein Implantat bereitgestellt, das aufgrund seiner oberflächlichen fluorhaltigen Schicht einen hydrophoben Charakter besitzt. Dadurch erweist sich das erfindungsgemäße Implantat mit besonderem Vorteil beständig gegenüber möglichen post-chirurgischen Gewebeverwachsungen, insbesondere im peritonealen Körperbereich.

Unter dem Begriff "fluorfreie Polymere" sollen Polymere verstanden werden, die aus fluorfreien Monomeren hergestellt sind.

In einer bevorzugten Ausführungsform sind die Fadenoberflächen, insbesondere alle Implantatfäden, vollflächig von der fluorhaltigen Schicht umgeben bzw. ummantelt. Eine vollflächige Umgebung bzw. Ummantelung der Fadenoberflächen mit der fluorhaltigen Schicht erhöht in besonders vorteilhafter Weise den hydrophoben und damit den gewebeadhäsionsbeständigen Charakter des Implantats.

In der fluorhaltigen Schicht ist Fluor kovalent an das Polymer des Implantats gebunden.

Weiterhin ist es erfindungsgemäß insbesondere vorgesehen, dass die Polymere, abgesehen von der fluorhaltigen Schicht, frei von Fluor sind.

Erfindungsgemäß kann es weiterhin vorgesehen sein, dass es sich bei dem Implantat um ein flächiges Implantat handelt.

In einer besonders bevorzugten Ausführungsform liegen die Fäden, die die fluorhaltige Schicht aufweisen, nur auf einer Flächenseite des Implantats vor. Bevorzugt handelt es sich bei dieser Seite um die Flächenseite des Implantats, welche nach Implantation dem Bauchraum zugewandt ist.

Grundsätzlich kann die fluorhaltige Schicht auf den Fadenoberflächen eine unterschiedliche Schichtdicke aufweisen. Bevorzugt weist die fluorhaltige Schicht auf den Fadenoberflächen jedoch eine einheitliche Schichtdicke auf. Erfindungsgemäß kann es insbesondere vorgesehen sein, dass die fluorhaltige Schicht 1 mg Fluor pro m² fluorierte Fadenoberfläche aufweist. Die fluorhaltige Schicht kann insbesondere entlang einer dünnen Übergangsschicht in das Implantat eindringen.

In einer weiteren Ausführungsform weist das Implantat ein Flächengewicht zwischen 36 und 70 g/cm² auf. Bevorzugt weist das Implantat ein Flächengewicht zwischen 36 und 45 g/cm², insbesondere 39 und 41 g/cm², auf. In bestimmten Fällen, insbesondere bei schwergewichtigen Patienten, können Flächengewichte zwischen 45 und 70 g/cm², insbesondere 55 und 65 g/cm², von Vorteil sein. Die in diesem Abschnitt aufgeführten Flächengewichte sind unter anderem deswegen vorteilhaft, da sie noch eine gewisse Flexibilität des Implantats ermöglichen, so dass dieses nach seiner Implantation auf im Körper auftretende mechanische Belastungen, beispielsweise durch angrenzendes Muskelgewebe verursachte Zugbelastungen, in angemessener Weise reagieren kann.

In einer weitergehenden Ausführungsform weist das Implantat Öffnungen mit einer lichten Öffnungsweite < 6000 µm, insbesondere zwischen 300 und 3500 µm, auf. Es ist weiterhin bevorzugt, dass das Implantat Öffnungen mit einer Gesamtfläche aufweist, die 50 bis 75 %, insbesondere 58 bis 70 %, der Grundfläche des Implantats entspricht. Insbesondere weist das Implantat Öffnungen auf, die frei von der fluorhaltigen Schicht sind. Dies bedeutet, dass die Implantatöffnungen in dieser Ausführungsform zumindest teilweise, vorzugsweise vollständig, nicht von der fluorhaltigen Schicht überzogen oder bedeckt sind.

Zweckmäßigerweise ist die fluorhaltige Schicht ablösefest mit den Fadenoberflächen verbunden. Eine ablösefeste Verbindung bedeutet in diesem Zusammenhang eine möglichst dauerhafte Verbindung mit den Fadenoberflächen des Implantats. Dies bedeutet insbesondere, dass sich die fluorhaltige Schicht nach einer Implantation nicht nach einer gewissen Zeit von den Implantaffäden wieder ablöst und dadurch in das umliegende Gewebe gelangt. Die fluorhaltige Schicht ist durch kovalente Bindungen mit den Fadenoberflächen verbunden. Die fluorhaltige Schicht selbst kann eine Fluorverbindung aufweisen oder aus einer solchen Verbindung bestehen. Bevorzugt sind in der fluorhaltigen Schicht Wasserstoffatome des Fadenmaterials zumindest zum Teil durch Fluoratome substituiert. Die fluorhaltige Schicht weist insbesondere fluorierte Alkylen- und/oder Alkylgruppen auf. Die Alkylen- bzw. Alkylgruppen können teil- und/oder perfluoriert sein. Bei den fluorierten Alkylen- bzw. Alkylgruppen handelt es sich vorzugsweise um CHF-, CF₂-, CH₂F-, CHF₂- und/oder CF₃-Gruppen.

Erfindungsgemäß kann es weiterhin bevorzugt sein, dass das Implantat einen Fluoratomanteil zwischen 1 und 10 Gew.-%, insbesondere 2 und 5 Gew.-%, bezogen auf das Gesamtgewicht des Implantats, aufweist. Der an sich geringe Fluoranteil bewirkt mit besonderem Vorteil keine im Hinblick auf mechanische oder elastische Eigenschaften nachteilige Gewichtserhöhung des Implantats.

Bei den Fäden des Implantats kann es sich erfindungsgemäß um mono- und/oder multifile Fäden handeln. Bevorzugt sind die Fäden monofil.

Als geeignetes Fadenmaterial kommen grundsätzlich alle fluorfreien Polymere in Frage. Zweckmäßigerweise sind die in Betracht kommenden Polymere bioverträglich. Bevorzugt ist das Fadenmaterial ein nicht resorbierbares Polymer. Das Fadenmaterial kann erfindungsgemäß ein Polyolefin, insbesondere Polyethylen und/oder Polypropylen, sein. Weiterhin können Polyester, insbesondere Polyethylenterephthalat, und/oder Polyamide als Fadenmaterialien verwendet werden.

Das Implantat ist ein textiles Implantat, insbesondere ein textiles Flächengebilde. Erfindungsgemäß kann es vorgesehen sein, dass es sich bei dem Implantat um ein Gewirk, Gestrick, Geflecht, Gewebe, Vlies oder Gelege handelt. Bevorzugt ist das Implantat ein Gewirk. Erfindungsgemäß ist es insbesondere vorgesehen, dass das Implantat ein textiles Netz, insbesondere ein Hernien-, Prolaps- oder Harninkontinenznetz, ist. Besonders bevorzugt liegt das erfindungsgemäße Implantat als Herniennetz vor. Bevorzugt ist das textile Netz ein Veloursnetz. Bezüglich weiterer Einzelheiten und Merkmale des Netzes wird auf die bisherige Beschreibung verwiesen.

Besonders bevorzugt weist das Implantat für Körperzellen hintergreifbare Stellen, insbesondere Veloursschlingen, Flottungen und/oder Flore, auf. Erfindungsgemäß kann es weiterhin vorgesehen sein, dass diese hintergreifbaren Stellen lediglich auf einer Seite, insbesondere Flächenseite, des Implantats vorliegen. Die hintergreifbaren Stellen können insbesondere als Verankerungsstrukturen für die Abdominalwand (Bauchwand) dienen.

Erfindungsgemäß kann es sich bei dem Implantat weiterhin um ein dreidimensionales Textilprodukt, insbesondere einen Plug, handeln. Das dreidimensionale Textilprodukt kann beispielsweise aus drei oder mehreren flächigen Textilgebilden aufgebaut sein. Die flächigen Textilgebilde können miteinander punktuell, linienförmig und/oder flächig verbunden, insbesondere verschweißt, sein. Erfindungsgemäß ist es insbesondere vorgesehen, dass zumindest ein flächiges Textilgebilde des dreidimensionalen Textilprodukts eine fluorhaltige Schicht aufweist.

Die vorliegende Erfindung betrifft weiterhin ein Verfahren zur Herstellung des medizinischen Implantats, wobei Fäden, insbesondere Fäden eines Implantats, zumindest teilweise mit einem Fluorgasgemisch behandelt werden, wodurch auf den Oberflächen der Fäden zumindest teilweise eine fluorhaltige Schicht erzeugt wird. Bei dem Fluorgasgemisch handelt es sich erfindungsgemäß um ein Gasgemisch, welches neben Fluor zumindest noch ein weiteres Gas aufweist.

In einer bevorzugten Ausführungsform wird zur Behandlung der Fäden ein Gasgemisch aus Fluor und Stickstoff, insbesondere in einem Fluor/Stickstoff-Volumenverhältnis von 1/100 bis 30/100, vorzugsweise 10/100, verwendet. Die Behandlung der Fäden wird vorzugsweise bei einem Druck von 10³ Pa bis 1,01325 x 10⁵ Pa (Atmosphärendruck), insbesondere 10⁴ Pa bis 5 x 10⁴ Pa, vorgenommen. Erfindungsgemäß kann es weiterhin vorgesehen sein, dass die Behandlung der Fäden in einem Temperaturbereich zwischen 0 °C und 90 °C, insbesondere 40 °C und 70 °C, durchgeführt wird. In einer weiteren Ausführungsform wird die Behandlung der Fäden während eines Zeitraums von 10 min bis 240 min, insbesondere 25 min bis 120 min, durchgeführt.

Gegebenenfalls können mehrere Fluorgasbehandlungszyklen gefahren werden, um beispielsweise die Schichtdicke der sich durch die Fluorgasgemischbehandlung auf den Fadenoberflächen bildenden fluorhaltigen Schicht zu verstärken.

Die behandelten Fäden werden zu einem textilen Implantat weiterverarbeitet. Die behandelten Fäden können miteinander gewirkt, gewebt, gestrickt oder geflochten werden. Weiterhin können die behandelten Fäden zu einem sogenannten nonwoven, insbesondere einem Vlies oder Gelege, verarbeitet werden. Diese textilen Verarbeitungstechniken für Fäden sind dem Fachmann hinreichend bekannt, so dass an dieser Stelle von weiteren Ausführungen abgesehen wird.

Wie bereits erwähnt, werden die Fäden erfindungsgemäß zumindest teilweise mit einem Fluorgasgemisch behandelt. Zu diesem Zweck können ein Teil der Fäden, insbesondere die Flächenseite eines Implantats, mit einem geeigneten Material, beispielsweise Silikon, abgedeckt werden. Eine anschließend durchgeführte Fluorgasgemisch-Behandlung führt lediglich zu einer Beschichtung der offenen, d.h. nicht abgedeckten, Fäden.

Auch alle medizinischen textilen Implantate, die nach einem erfindungsgemäßen Verfahren hergestellt oder herstellbar sind, sind Gegenstand dieser Erfindung.

Die Erfindung betrifft schließlich auch die Verwendung des medizinischen Implantats in der visceralen und/oder parietalen Chirurgie, insbesondere zur Versorgung von Hernien.

Weitere Einzelheiten und Merkmale der Erfindung ergeben sich aus der nachfolgenden Beschreibung bevorzugter Ausführungsformen in Form von Beispielen in Kombination mit den Unteransprüchen. In diesen Ausführungsformen können einzelne Merkmale der Erfindung allein oder in Kombination mit anderen Merkmalen verwirklicht sein. Die beschriebenen bevorzugten Ausführungsformen sind lediglich als beschreibende, keineswegs als in irgendeiner Weise limitierende Offenbarung zu verstehen.

### Beispiel 1:

Fluorierung von Optilene^{®} Mesh LP (leichtgewichtiges Netz aus Polypropylen-Fäden mit einem Flächengewicht von ca. 38,1 g/m² und einer Porengröße von ca. 1 mm)

Das Optilene^{®} Netz LP wird auf eine Größe von ca. 36,5 cm x 36,1 cm zurechtgeschnitten und anschließend in einen Fluorierungsreaktor eingebracht. Vor der eigentlichen Behandlung des Netzes werden zwei Reinigungszyklen mit reinem Stickstoff in einem Druckbereich zwischen 10³ Pa und 8 x 10⁴ Pa gefahren. Danach wird das Netz bei einem Stickstoffdruck von etwa 5 x 10⁴ Pa und während einer Zeitdauer von etwa 300 Sekunden in dem Reaktor vorgewärmt. Zur Fluorierung des Netzes wird ein Fluorgasgemisch aus Fluor und Stickstoff, das gegebenenfalls auch technischen Stickstoff enthalten kann, verwendet. Der Anteil von Fluor im Behandlungsgas beträgt etwa 10 Vol.-%. Die Fluorierung des Netzes wird bei einem Gasdruck von etwa 3,5 x 10⁴ Pa und während einer Zeitdauer von etwa 3600 Sekunden vorgenommen. Anschließend werden zwei Reinigungszyklen gefahren, um Restgase aus dem Reaktor zu entfernen.

Das behandelte Netz wies rein äußerlich keine Veränderungen auf, insbesondere keine farblichen Veränderungen. Die Auswaage des behandelten Netzes ergab eine geringfügige Gewichtszunahme von etwa 4,01 % gegenüber dem unbehandelten Netz. Dies zeigt, dass die Leichtgewichtigkeit des Netzes durch die Fluorierung im Wesentlichen unberührt bleibt.

### Beispiel 2:

### Herstellung eines Optilene^{®} Mesh LP, das nur auf einer Flächenseite fluoriert ist

Ein leichtgewichtiges Optilene^{®} Netz LP (vgl. Beispiel 1) wird, wie in Beispiel 1 beschrieben, in einen Fluorierungsreaktor gehängt, wobei eine Flächenseite des Netzes mit einer Silikonmatte abgedeckt wird. Anschließend erfolgt eine Fluorierung des Netzes entsprechend Beispiel 1. Nach Beendigung der Fluorierungsbehandlung wird die Silikonmatte entfernt und das Netz mit einem Lösungsmittelgemisch aus 2-Propanol und Aceton (2-Propanol/Aceton 70/30 (v/v) gewaschen. Das auf diese Weise erhaltene Netz ist lediglich auf seiner unbedeckten Flächenseite fluoriert.

### Beispiel 3:

### Überprüfung im Tierversuch

Zur Durchführung der Tierversuche wurden acht weibliche SPF-Albino-Kaninchen der Rasse Chbb: HM(SPF)-Kleinrusse (Charles River Deutschland GmbH) verwendet. Die Kaninchen hatten ein Körpergewicht zwischen 2,4 und 3,2 kg und wurden mit National Wing Band Ohrmarken gekennzeichnet. Zur Vorbereitung der Implantation wurden die Kaninchen anestisiert und anschließend deren Blinddarm (Caecum) traumatisiert. Danach wurde der Blinddarm mittels einer sterilen Gazekompresse für etwa 3,5 Minuten traumatisiert, wobei punktförmige bis flächige leichte Blutungen auf der Oberfläche des Blinddarms auftraten. Anschließend wurde ein etwa 2,5 cm x 2,5 cm großes Stück des Peritoneums mit dem darunter liegenden Musculus transversus abdominalis aus der rechten Bauchwanddeckenseite entfernt. Ein entsprechend großes Netzstück (3,5 cm x 3,5 cm) wurde auf den Bauchwanddefekt aufgelegt und mit einer umlaufenden Naht (Nahtmaterial Resopren^{®}) fixiert.

Vier Kaninchen wurde auf diese Weise ein gemäß Beispiel 1 behandeltes Optilene^{®} Netz implantiert. Die übrigen vier Kaninchen erhielten ein unbehandeltes leichtgewichtiges Optilene^{®} Netz. Die Tiere wurden 21 Tage nach dem Eingriff getötet, seziert und makroskopisch bezüglich des Verwachsungsanteils der explantierten Netze beurteilt. Hierbei ergaben sich folgende Ergebnisse:

| | Tier Nr. | Adhäsionen in % zur Gesamtfläche des Bauchwanddefektes |
|---|---|---|
| Netz (entsprechend Beispiel 1 behandelt) | 3091 | 30 |
| | 3097 | frei |
| | 3123 | 40 |
| | 3165 | frei |
| Netz (unbehandelt) | 3185 | 90 |
| | 3391 | 85 |
| | 3362 | 100 |
| | 3415 | 95 |

Die Ergebnisse zeigen deutlich, dass die fluorierten Netze im Vergleich zu den unfluorierten Netzen eine signifikante Verringerung von post-chirurgischen Gewebeverklebungen bewirken. Zwei der explantierten fluorierten Herniennetze wiesen überhaupt keine post-chirurgischen Gewebeverwachsungen auf.

## Patentansprüche

1. Medizinisches Implantat aus an sich fluorfreien Polymeren, insbesondere zur Versorgung von Hernien, wobei das Implantat zumindest teilweise Fäden aufweist, die an ihrer Oberfläche zumindest teilweise eine fluorhaltige Schicht aufweisen, **dadurch gekennzeichnet, dass** in der fluorhaltigen Schicht Fluor kovalent an das Polymer des Implantats gebunden ist und das Implantat ein textiles Implantat ist.

2. Implantat nach Anspruch 1, **dadurch gekennzeichnet, dass** die Polymere, abgesehen von der fluorhaltigen Schicht, frei von Fluor sind.

3. Implantat nach einem der vorhergehenden Ansprüche, **dadurch gekennzeichnet, dass** die Fäden, die die fluorhaltige Schicht aufweisen, nur auf einer Flächenseite des Implantats vorliegen.

4. Implantat nach einem der vorhergehenden Ansprüche, **dadurch gekennzeichnet, dass** die fluorhaltige Schicht 1 mg Fluor pro m² fluorierte Fadenoberfläche aufweist.

5. Implantat nach einem der vorhergehenden Ansprüche, **dadurch gekennzeichnet, dass** das Implantat Öffnungen aufweist, die frei von der fluorhaltigen Schicht sind.

6. Implantat nach einem der vorhergehenden Ansprüche, **dadurch gekennzeichnet, dass** die fluorhaltige Schicht fluorierte Alkylen- und/oder Alkylgruppen aufweist, vorzugsweise CHF-, CF₂-, CH₂F-, CHF₂-und/oder CF₃-Gruppen.

7. Implantat nach einem der vorhergehenden Ansprüche, **dadurch gekennzeichnet, dass** das Implantat einen Fluoratomanteil zwischen 1 und 10 Gew.-%, insbesondere 2 und 5 Gew.-%, bezogen auf das Gesamtgewicht des Implantats, aufweist.

8. Implantat nach einem der vorhergehenden Ansprüche, **dadurch gekennzeichnet, dass** das Implantat ein textiles Netz ist.

9. Implantat nach einem der vorhergehenden Ansprüche, **dadurch gekennzeichnet, dass** das Implantat für Körperzellen hintergreifbare Stellen, insbesondere Veloursschlingen, Flottungen und/oder Flore, aufweist.

10. Verfahren zur Herstellung eines medizinischen Implantats nach einem der vorhergehenden Ansprüche, **dadurch gekennzeichnet, dass** Fäden zumindest teilweise mit einem Fluorgasgemisch behandelt werden, wodurch auf den Fadenoberflächen zumindest teilweise eine fluorhaltige Schicht erzeugt wird.

11. Verfahren nach Anspruch 10, **dadurch gekennzeichnet, dass** zur Behandlung der Fäden ein Gasgemisch aus Fluor und Stickstoff, insbesondere in einem Fluor/Stickstoff-Volumen-Verhältnis von 1/100 bis 30/100, vorzugsweise 10/100, verwendet wird.

12. Verfahren nach Anspruch 10 oder 11, **dadurch gekennzeichnet, dass** die Behandlung der Fäden bei einem Druck von 10³ Pa bis 1,01325 x 10⁵ Pa, insbesondere 10⁴ Pa bis 5 x 10⁴ Pa, vorgenommen wird.

13. Medizinisches Implantat, herstellbar nach einem Verfahren nach einem der vorhergehenden Ansprüche 10 bis 12.

14. Implantat nach einem der Ansprüche 1 bis 9 zur Verwendung in der visceralen und/oder parietalen Chirurgie, insbesondere zur Versorgung von Hernien.

## Claims

1. Medical implant composed of polymers which are fluorine-free per se, in particular for the management of hernias, where the implant has at least in part threads having on their surface at least in part a fluorine-containing layer, **characterized in that**, in the fluorine-containing layer, fluorine is covalently bonded to the polymer of the implant and the implant is a textile implant.

2. Implant according to Claim 1, **characterized in that** the polymers, apart from the fluorine-containing layer, are free of fluorine.

3. Implant according to either of the preceding claims, **characterized in that** the threads having the fluorine-containing layer are present on only one surface side of the implant.

4. Implant according to any of the preceding claims, **characterized in that** the fluorine-containing layer has 1 mg of fluorine per m² of fluorinated thread surface.

5. Implant according to any of the preceding claims, **characterized in that** the implant has apertures which are free of the fluorine-containing layer.

6. Implant according to any of the preceding claims, **characterized in that** the fluorine-containing layer has fluorinated alkylene and/or alkyl groups, preferably CHF, CF₂, CH₂F, CHF₂ and/or CF₃ groups.

7. Implant according to any of the preceding claims, **characterized in that** the implant has a fluorine atom content of between 1 and 10% by weight, in particular 2 and 5% by weight, based on the total weight of the implant.

8. Implant according to any of the preceding claims, **characterized in that** the implant is a textile mesh.

9. Implant according to any of the preceding claims, **characterized in that** the implant has sites engageable by body cells, in particular velour loops, floats and/or pile warps.

10. Process for producing a medical implant according to any of the preceding claims, **characterized in that** threads are treated at least partly with a fluorine gas mixture, by which means a fluorine-containing layer is generated on at least part of the thread surfaces.

11. Process according to Claim 10, **characterized in that** a gas mixture of fluorine and nitrogen, in particular in a fluorine/nitrogen ratio by volume of 1/100 to 30/100, preferably 10/100, is used for treating the threads.

12. Process according to Claim 10 or 11, **characterized in that** the treatment of the threads is carried out under a pressure of from 10³ Pa to 1.01325 x 10⁵ Pa, in particular 10⁴ Pa to 5 x 10⁴ Pa.

13. Medical implant which can be produced by a process according to any of the preceding Claims 10 to 12.

14. Implant according to any of Claims 1 to 9 for use in visceral and/or parietal surgery, in particular for the management of hernias.

## Revendications

1. Implant médical en polymères en soi exempts de fluor, en particulier pour le soin de hernies, l'implant présentant au moins partiellement des fils, qui présentent, en leur surface, au moins partiellement une couche fluorée, **caractérisé en ce que** dans la couche contenant du fluor, du fluor est lié par covalence au polymère de l'implant et l'implant est un implant textile.

2. Implant selon la revendication 1, **caractérisé en ce que** les polymères, en dehors de la couche contenant du fluor, sont exempts de fluor.

3. Implant selon l'une quelconque des revendications précédentes, **caractérisé en ce que** les fils qui présentent la couche contenant du fluor, ne se trouvent que sur une face de l'implant.

4. Implant selon l'une quelconque des revendications précédentes, **caractérisé en ce que** la couche contenant du fluor contient 1 mg de fluor par m² de surface de fil fluoré.

5. Implant selon l'une quelconque des revendications précédentes, **caractérisé en ce que** l'implant présente des ouvertures, qui sont exemptes de la couche contenant du fluor.

6. Implant selon l'une quelconque des revendications précédentes, **caractérisé en ce que** la couche contenant du fluor présente des groupes alkylène et/ou alkyle fluorés, de préférence des groupes CHF, CF₂, CH₂F, CHF₂ et/ou CF₃.

7. Implant selon l'une quelconque des revendications précédentes, **caractérisé en ce que** l'implant présente une proportion d'atomes de fluor entre 1 et 10% en poids, en particulier entre 2 et 5% en poids, par rapport au poids total de l'implant.

8. Implant selon l'une quelconque des revendications précédentes, **caractérisé en ce que** l'implant est un filet textile.

9. Implant selon l'une quelconque des revendications précédentes, **caractérisé en ce que** l'implant présente des sites derrière lesquels les cellules corporelles peuvent s'agripper, en particulier des boucles en velours, des fils flottants et/ou des poils.

10. Procédé pour la réalisation d'un implant médical selon l'une quelconque des revendications précédentes, **caractérisé en ce que** les fils sont au moins partiellement traités par un mélange de gaz fluorés, suite à quoi une couche contenant du fluor est au moins partiellement générée sur les surfaces des fils.

11. Procédé selon la revendication 10, **caractérisé en ce qu'**on utilise, pour le traitement des fils, un mélange gazeux de fluor et d'azote, en particulier dans un rapport volumique fluor/azote de 1/100 à 30/100, de préférence de 10/100.

12. Procédé selon la revendication 10 ou 11, **caractérisé en ce que** le traitement des fils est réalisé à une pression de 10³ Pa à 1,01325 x 10⁵ Pa, en particulier de 10⁴ Pa à 5 x 10⁴ Pa.

13. Implant médical, pouvant être produit par un procédé selon l'une quelconque des revendications précédentes 10 à 12.

14. Implant selon l'une quelconque des revendications 1 à 9 destiné à une utilisation en chirurgie viscérale et/ou pariétale, en particulier pour le soin de hernies.
